# EUROPEAN PATENT SPECIFICATION

(11) **EP 4 047 365 B1**
(45) Date of publication and mention of the grant of the patent: **10.05.2023**
(21) Application number: 22020144.6
(22) Date of filing: 01.04.2019
(51) Int. Cl.: G01N 33/22, B67D 7/04, B67D 7/34

(54) **DEVICE FOR DETECTING FUEL TYPE FOR FUEL TRANSFER**
VORRICHTUNG ZUR ERKENNUNG DES KRAFTSTOFFTYPS ZUR KRAFTSTOFFÜBERTRAGUNG
DISPOSITIF DE DÉTECTION DU TYPE DE CARBURANT POUR LE TRANSFERT DE CARBURANT

(30) Priority: 13.04.2018 TR 201805287
(43) Date of publication of application: 24.08.2022
(62) Divisional of application: 19815001.3
(73) Proprietor: Asis Otomasyon Ve Akaryakit Sistemleri Anonim Sirketi, 34775 Ümraniye/Istanbul (TR)
(72) Inventor: Kaya, Yusuf, 34775 Ümraniye/Istanbul (TR)

(56) References cited:
- US-A- 5 172 738
- US-A- 5 209 275
- US-A- 6 068 030

## Description

### TECHNICAL FIELD

The present invention relates to a device for detecting fuel type, which may be used for controlling transfer of fuel from one location to another. In particular, the invention relates to a device configured to detect the fuel type from its gaseous form.

### BACKGROUND OF THE INVENTION

Current data estimates that there are globally about 1.2 billion motor vehicles using fossil fuel. This number brings about hundred thousands of active fuel filling and refueling stations. This increasing number and the variety in fuel types require meticulous management of transferring fuel from one location to another. Indeed, engines of vehicles such as land vehicles working on fossil fuel are designed to use a certain type of fuel. It is known that an engine not supplied with the suitable type of fuel would break down, therefore various solutions have been suggested over the years against "wrong fuel feeding" or "cross filling."

For example, US 4838323A describes that the control between two types of fuel such as gasoline and diesel fuel may be realized by a sensor system that can detect the difference in vapor pressure between them and a fuel cut-off apparatus that can communicate with such system. According to US4838323A, said sensor system can make detection by sampling the fuel inside the fuel tank of an automobile.

US 6341629A discloses a method for controlling the movement of a liquid from a first location to a second location via a dispenser for the liquid located in a liquid line between the first and second location wherein the dispenser includes a nozzle, an internal liquid conduit, a valve, a body portion, a hollow annular collar and a first detector. The first detector is located in the collar which is positioned around the nozzle or between the nozzle and the body portion such that the first detector is in vapor communication with a vapor space above the second location. The vapor from the vapor space is analyzed by withdrawing the vapor past the first detector and using the results of the analysis to control the operation of the valve, thereby determining as to whether fuel transfer will be made or not.

US 5209275A relates to a fuel dispensing apparatus which includes a detecting element for detecting the type of fuel from the gas vaporized from such fuel remaining in a storage tank to be supplied with fuel.

On the other hand, wrong fuel transfer can occur not only between a fuel dispenser and an automobile but also during the refueling of underground tanks at gas stations which can cause extremely inconvenient consequences such as total cleaning of the underground tank. It is also known that other solutions aiming to detect the type of the fuel in liquid form have been generated for such circumstances. However, these solutions have not sufficiently extended through the marked due to the high costs they entail.

### SUMMARY OF THE INVENTION

The object of the invention is to effectively detect the fuel type.

Further object of the invention is to effectively prevent faulty fuel transfer from a first location to a second location.

In line with the objects, the present invention relates to a device for controlling the transfer of fuel from a first location to a second location, comprising a sensor device for detecting the fuel type in a location and a controller communicating with the sensor device, characterized in that said sensor device comprises a first chamber and a second chamber, at least a pored membrane arranged to separate the chambers, a gas intake associated with the first chamber for receiving a gas form of the fuel, a gas outlet associated with the second chamber for discharging a gas form of the fuel, at least one first gas sensor communicating with said first chamber and at least one second gas sensor communicating with said second chamber.

According to the invention, the size of the pores of the membrane is selected such that the fuel gas molecules bigger than the pore size of the membrane are retained in the first chamber and the fuel gas molecules smaller than the pore size of the membrane are passed to the second chamber. Since each chamber is provided with a respective gas sensor, the density of the fuel gas molecules in each chamber is measured/detected and then compared by the controller.

### BRIEF DESCRIPTION OF THE DRAWINGS

The present invention should be evaluated in line with the figures described below in order to attain the best possible understanding of its configuration and advantages in combination with its supplementary elements.
Fig. 1 is a symbolic cross-sectional side view of the device with a pored membrane.
Fig. 2 is a symbolic cross-sectional side view of the device with a pored membrane.

### REFERENCE NUMBERS OF THE ELEMENTS SHOWN IN THE FIGURES

- 11: Sensor device
- 17: First gas sensor
- 18: Second gas sensor
- 19: Gas intake
- 20: Gas outlet
- 21: Membrane
- 22: Pore
- 23: Diesel molecules
- 24: Gasoline molecules
- 25: First chamber
- 26: Second chamber

### DETAILED DESCRIPTION OF THE INVENTION

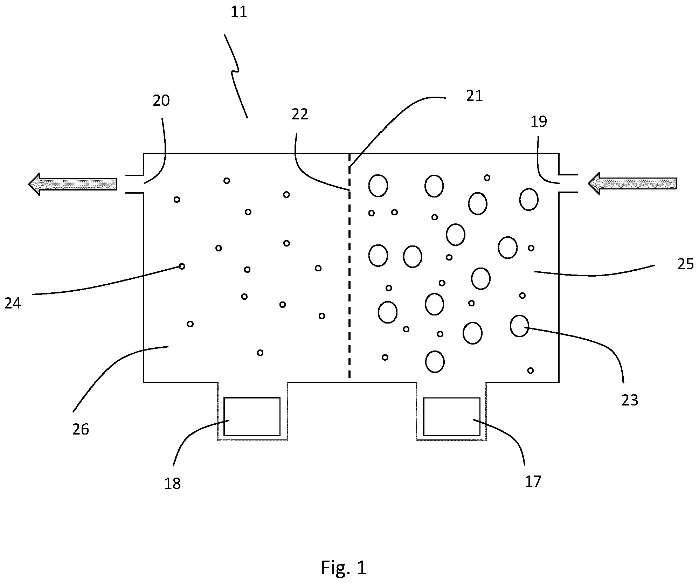

According to an embodiment of the invention, the fuel transfer control between a first location and a second location is provided by a pored membraned sensor device. Fig. 1 shows an example for such a sensor device. The sensor device (11) defines a structure in substantially closed form containing a gas intake (19) and a gas outlet (20), the gas intake (19) and the gas outlet (20) preferably oppose each other. Inside said sensor device (11), there is an empty chamber divided into two sections through a pored membrane (21) to define a first chamber (25) and a second chamber (26).

The gas intake (19) is configured at the side of the first chamber (25) and the gas outlet (20) is configured at the side of the second chamber (26). The configuration of the membrane (21) comprises a frame which preferably comprises micro-scale pores (22) of 0.10-0.50 µm and one or more polymer layers accumulated thereon. As the microporous frame, various materials may be used such as polysulfone (PS), PI, polyetherimide (PEI), polyvinylidene fluoride (PVDF) or sulfonated tetrafluoroethylene fluoropolymer-copolymer (Nafion^{®}). As the polymeric layer, various materials may be used such as polyether block amide (PEBA), polyethylene glycol (PEG), polyimide (PI), polyurethane (PU) or polydimethylsiloxane (PDMS). For enhancing the mechanical resistance and selectivity of the membrane, cross-linking within the polymeric layer or interlayer cross-linking may be applied. Cross-linking may be enabled through agents such as maleic anhydride, glutaraldehyde, etc. and also by heat treatment. The selectivity and gas permeation rates of the membrane may be adjusted by using inorganic nano-fillers on polymer layers, such as graphene oxide or carbon nanotubes.

The polymers in the quantity determined for the production of the membrane according to the invention may be dissolved by using appropriate solvents such as isopropanol/n-propanol, n-butanol or hexane. If it is aimed to use nano-fillers, then the selected inorganic material may be dispersed in this solution. The polymer-inorganic material mixture may be cast by using a membrane casting apparatus in order to prepare a microporous frame structure of the desired thickness.

The accumulation process is performed on the selected microporous frame. Parameters such as the duration of the process, selection of the solvent medium, need for a catalyst and the solvent removal step may be determined depending on the frame and the polymer.

The synthesized membranes may be subjected to various functionality tests such as mechanical and thermal resistance and gas permeability, thereby determining the selectivity values.

The size of the pores (22) of the membrane (21) will permit fuel gas molecules of certain molecular size to permeate whereas larger molecules will not be permitted. As shown in Fig. 1, when diesel gas enters into the gas intake, since diesel molecules (23) will not permeate through the membrane pores (22), they will accumulate in the first chamber (25). As is known, diesel gas may not always wholly comprise diesel molecules and may also have smaller molecules, i.e. molecules of the size of gasoline molecules (24) even at small rates, a portion of which will permeate through the membrane pores (22) and accumulate in the second chamber (26). Accordingly, the membrane (21) will separate gas molecules of different sizes so that they can be kept in the first chamber (25) and the second chamber (26), respectively.

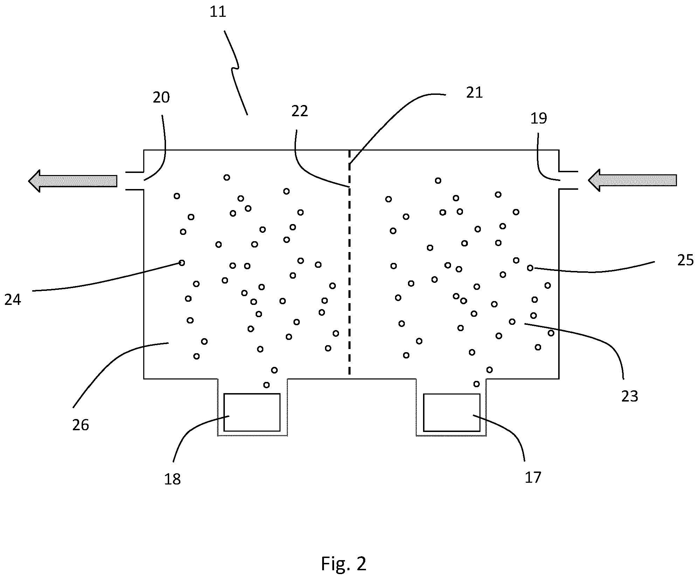

The first chamber (25) is provided with a first gas sensor (17) and the second chamber (26) is provided with a second gas sensor (18). The first gas sensor (17) and the second gas sensor (18) may for example be a known VOC sensor or MEMS metal oxide sensor or air quality sensor or MEMS sensor. Said sensors (17, 18) communicate with a control unit. In practice, the signal received from the first gas sensor (17) (the signal related to the gas density level) and the signal received from the second gas sensor (18) (the signal related to the gas density level) are compared and interpreted by the control unit which is coupled to a dispenser for fuel transfer. If the signal received from the first gas sensor (17) is different from the signal received from the second gas sensor (18) on the basis of a predetermined tolerance range and if the rate between said signals is within a tolerance range determined for diesel vapor, then it may be decided that the gas mixture entering into the sensor device (11) is a large-molecule gas mixture formed by the vaporization of diesel. For example, as shown in Fig. 2, if there is a specific rate between the signal received from the first gas sensor (17) and the signal received from the second gas sensor (18) based on the predetermined tolerance range for gasoline vapor, then it may be decided that the gas mixture entering into the sensor device (11) is a gas mixture formed by the vaporization of gasoline.

According to an embodiment of the invention, a suction fan may be arranged at the gas outlet (20) side, thereby enabling a more regular and rapid exit of the gas entering through the gas intake (19) from the sensor device (11) chamber.

According to an embodiment of the invention, a suction fan may be arranged at the gas intake (19) side, thereby enabling a more regular and rapid introduction of the gas through the gas intake (19) inside the sensor device (11) chamber.

According to an embodiment of the invention, the fan arranged at the gas intake (19) side may enable both a more regular and rapid introduction of the gas through the gas intake (19) inside the sensor device (11) chamber and also rapid discharge of the fuel vapor entering into the sensor device (11) chamber through the gas intake (19) again from the intake (19).

## Claims

1. A device for controlling the transfer of fuel from a first location to a second location, comprising a sensor device (11) for detecting the fuel type in a location and a controller communicating with the sensor device (11), **characterized in that** said sensor device (11) comprises a first chamber (25) and a second chamber (26), at least a pored membrane (21) arranged to separate the chambers (25, 26), a gas intake (19) connected to the first chamber (25) for receiving a gas form of the fuel, a gas outlet (20) associated with the second chamber (26) for discharging a gas form of the fuel, at least one first gas sensor (17) communicating with said first chamber (25) and at least one second gas sensor (18) communicating with said second chamber (26).

2. The device according to Claim 1, wherein the pored membrane (21) comprises a microporous frame and one or more polymer layers accumulated thereon.

3. The device according to Claim 2, wherein the microporous frame is selected from the group consisting of polysulfone (PS), PI, polyetherimide (PEI), polyvinylidene fluoride (PVDF) and sulfonated tetrafluoroethylene fluoropolymer-copolymer (Nafion^{®}).

4. The device according to Claim 2, wherein said polymer is selected from the group consisting of polyether block amide (PEBA), polyethylene glycol (PEG), polyimide (PI), polyurethane (PU) and polydimethylsiloxane (PDMS).

5. The device according to Claim 4, wherein said polymer comprises cross-linking by an agent selected from the group consisting of maleic anhydride and glutaraldehyde.

6. The device according to Claim 4, wherein said polymer comprises an inorganic nano-filler selected from the group consisting of graphene oxide and carbon nanotubes.

7. The device according to Claim 1, wherein said at least one first gas sensor (17) and said at least one second gas sensor (18) are selected from the group consisting of a VOC sensor, MEMS metal oxide sensor, air quality sensor and MEMS sensor.

8. The device according to Claim 1, wherein the device comprises at least one fan.

9. The device according to Claim 8, wherein the device comprises at least one suction fan provided at the side of said gas intake (19) and/or at the side of said gas outlet (20).

10. The device according to anyone of the preceding claims, wherein said fuel is gasoline or diesel fuel.

## Patentansprüche

1. Eine Vorrichtung zum Steuern der Übertragung von Kraftstoff von einem ersten Ort an einen zweiten Ort, beinhaltend eine Sensorvorrichtung (11) zum Detektieren des Kraftstofftyps an einem Ort und eine Steuereinheit, die mit der Sensorvorrichtung (11) kommuniziert, **dadurch gekennzeichnet, dass** die Sensorvorrichtung (11) Folgendes beinhaltet: eine erste Kammer (25) und eine zweite Kammer (26), mindestens eine mit Poren versehene Membran (21), die eingerichtet ist, um die Kammern (25, 26) zu trennen, eine Gasaufnahme (19), die mit der ersten Kammer (25) verbunden ist, um eine Gasform des Kraftstoffs zu empfangen, einen Gasauslass (20), der mit der zweiten Kammer (26) assoziiert ist, um eine Gasform des Kraftstoffs abzulassen, mindestens einen ersten Gassensor (17), der mit der ersten Kammer (25) kommuniziert, und mindestens einen zweiten Gassensor (18), der mit der zweiten Kammer (26) kommuniziert.

2. Vorrichtung gemäß Anspruch 1, wobei die mit Poren versehene Membran (21) einen mikroporösen Rahmen und eine oder mehrere darauf abgelagerte Polymerschichten beinhaltet.

3. Vorrichtung gemäß Anspruch 2, wobei der mikroporöse Rahmen aus der Gruppe ausgewählt ist, die aus Polysulfon (PS), PI, Polyetherimid (PEI), Polyvinylidenfluorid (PVDF) und sulfoniertem Tetrafluorethylen-Fluorpolymer-Copolymer (Nafion^{®}) besteht.

4. Vorrichtung gemäß Anspruch 2, wobei das Polymer aus der Gruppe ausgewählt ist, die aus Polyetherblockamid (PEBA), Polyethylenglycol (PEG), Polyimid (PI), Polyurethan (PU) und Polydimethylsiloxan (PDMS) besteht.

5. Vorrichtung gemäß Anspruch 4, wobei das Polymer Vernetzung durch ein Mittel beinhaltet, das aus der Gruppe ausgewählt ist, die aus Maleinsäureanhydrid und Glutaraldehyd besteht.

6. Vorrichtung gemäß Anspruch 4, wobei das Polymer einen anorganischen Nanofüllstoff beinhaltet, der aus der Gruppe ausgewählt ist, die aus Graphenoxid und Kohlenstoffnanoröhrchen besteht.

7. Vorrichtung gemäß Anspruch 1, wobei der mindestens eine erste Gassensor (17) und der mindestens eine zweite Gassensor (18) aus der Gruppe ausgewählt sind, die aus einem VOC-Sensor, einem MEMS-Metalloxidsensor, einem Luftqualitätssensor und einem MEMS-Sensor besteht.

8. Vorrichtung gemäß Anspruch 1, wobei die Vorrichtung mindestens ein Gebläse beinhaltet.

9. Vorrichtung gemäß Anspruch 8, wobei die Vorrichtung mindestens ein Sauggebläse beinhaltet, das an der Seite der Gasaufnahme (19) und/oder an der Seite des Gasauslasses (20) bereitgestellt ist.

10. Vorrichtung gemäß einem der vorhergehenden Ansprüche, wobei der Kraftstoff Benzin oder Dieselkraftstoff ist.

## Revendications

1. Un dispositif pour commander le transfert de carburant d'un premier emplacement à un deuxième emplacement, comprenant un dispositif capteur (11) pour détecter le type de carburant dans un emplacement et un contrôleur communiquant avec le dispositif capteur (11), **caractérisé en ce que** ledit dispositif capteur (11) comprend une première chambre (25) et une deuxième chambre (26), au moins une membrane à pores (21) agencée pour séparer les chambres (25, 26), une admission de gaz (19) raccordée à la première chambre (25) pour recevoir une forme gazeuse du carburant, une sortie de gaz (20) associée à la deuxième chambre (26) pour décharger une forme gazeuse du carburant, au moins un premier capteur de gaz (17) communiquant avec ladite première chambre (25) et au moins un deuxième capteur de gaz (18) communiquant avec ladite deuxième chambre (26).

2. Le dispositif selon la revendication 1, dans lequel la membrane à pores (21) comprend un cadre microporeux et une ou plusieurs couches de polymère accumulées sur celuici.

3. Le dispositif selon la revendication 2, dans lequel le cadre microporeux est sélectionné dans le groupe constitué de polysulfone (PS), de Pl, de polyétherimide (PEI), de fluorure de polyvinylidène (PVDF) et de copolymère de tétrafluoroéthylène fluoropolymère (Nafion^{®}).

4. Le dispositif selon la revendication 2, dans lequel ledit polymère est sélectionné dans le groupe constitué de polyéther bloc amide (PEBA), de polyéthylène glycol (PEG), de polyimide (PI), de polyuréthane (PU) et de polydiméthylsiloxane (PDMS).

5. Le dispositif selon la revendication 4, dans lequel ledit polymère comprend une réticulation par un agent sélectionné dans le groupe constitué d'anhydride maléique et de glutaraldéhyde.

6. Le dispositif selon la revendication 4, dans lequel ledit polymère comprend une nano-charge inorganique sélectionnée dans le groupe constitué d'oxyde de graphène et de nanotubes de carbone.

7. Le dispositif selon la revendication 1, dans lequel ledit au moins un premier capteur de gaz (17) et ledit au moins un deuxième capteur de gaz (18) sont sélectionnés dans le groupe constitué d'un capteur COV, d'un capteur d'oxyde de métal MEMS, d'un capteur de qualité de l'air et d'un capteur MEMS.

8. Le dispositif selon la revendication 1, le dispositif comprenant au moins un ventilateur.

9. Le dispositif selon la revendication 8, le dispositif comprenant au moins un ventilateur aspirant fourni au niveau du côté de ladite admission de gaz (19) et/ou au niveau du côté de ladite sortie de gaz (20).

10. Le dispositif selon n'importe laquelle des revendications précédentes, dans lequel ledit carburant est de l'essence ou du gasoil.
